(19) **Europäisches Patentamt / European Patent Office / Office européen des brevets**

(11) **EP 4 374 779 B1**

(12) **EUROPEAN PATENT SPECIFICATION**

(45) Date of publication and mention of the grant of the patent:
**04.06.2025  Bulletin 2025/23**

(21) Application number: **23170328.1**

(22) Date of filing: **27.04.2023**

(51) International Patent Classification (IPC):
**A61B 5/01** *(2006.01)*    **A61B 5/026** *(2006.01)*

(52) Cooperative Patent Classification (CPC):
**A61B 5/028; A61B 5/015; A61B 5/026; A61B 5/681; A61B 5/6831; A61B 5/6833; A61B 5/14532**

(54) **ELECTRONIC DEVICE AND METHOD OF ESTIMATING BLOOD FLOW INFORMATION USING THE SAME**

ELEKTRONISCHE VORRICHTUNG UND VERFAHREN ZUR SCHÄTZUNG VON BLUTFLUSSINFORMATIONEN

DISPOSITIF ÉLECTRONIQUE ET PROCÉDÉ D'ESTIMATION D'INFORMATIONS DE DÉBIT SANGUIN

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **25.11.2022  KR 20220160847**

(43) Date of publication of application:
**29.05.2024  Bulletin 2024/22**

(73) Proprietors:
- **Samsung Electronics Co., Ltd.
  Suwon-si, Gyeonggi-do 16677 (KR)**
- **Korea Advanced Institute of Science and Technology
  Daejeon 34141 (KR)**

(72) Inventors:
- **KWON, Bok Soon
  Suwon-si, Gyeonggi-do (KR)**
- **KWON, Kyeongha
  Daejeon (KR)**
- **PARK, Do Yun
  Seoul (KR)**
- **SIM, Young Min
  Daejeon (KR)**
- **JUNG, Chulyoon
  Daejeon (KR)**
- **KIM, Sang Kyu
  Suwon-si, Gyeonggi-do (KR)**
- **KIM, Sungho
  Suwon-si, Gyeonggi-do (KR)**
- **LEE, So Young
  Suwon-si, Gyeonggi-do (KR)**
- **LEE, Ho Taik
  Suwon-si, Gyeonggi-do (KR)**
- **RHEE, Hong Soon
  Suwon-si, Gyeonggi-do (KR)**

(74) Representative: **Grünecker Patent- und Rechtsanwälte
PartG mbB
Leopoldstraße 4
80802 München (DE)**

(56) References cited:
**US-A1- 2012 296 224    US-A1- 2018 014 734
US-A1- 2019 388 031    US-A1- 2022 128 413**

**Description**

BACKGROUND

**1. Field**

Apparatuses and method according to one or more embodiments relate to enabling obtaining blood flow information, such as blood flow velocity, by using the electronic device.

**2. Description of the Related Art**

Blood flow is an important physiological parameter that can provide valuable information about a person's cardiovascular health, such as stroke, hypertension, hyperlipidemia, and the like. Conventional methods for measuring blood flow, such as blood pressure cuffs or vascular ultrasonography, are invasive and/or bulky, making them unsuitable for daily use and for continuous monitoring. Accordingly, there is an increasing need for a wearable device capable of accurately and continuously measuring blood flow in daily life, which would provide valuable insights into a person's cardiovascular health and potentially lead to early detection and treatment of cardiovascular disease.

US 2022/0128413 A1 concerns a core body temperature sensor for measuring the core body temperature of a body in a non-invasive way via applying the core body temperature sensor to a surface of the bod. The core body temperature sensor comprises: at least a first thermistor pair of opposing thermistors across a first thermal insulator and a second thermistor pair, adjacent to the first thermistor pair, of opposing thermistors across a second thermal insulator, and a means to measure blood perfusion.

US 2019/0388031 A1 relates to techniques for non-invasive core body temperature monitoring. A core body temperature monitoring apparatus placed superdermally over a user's skin, including a first temperature sensor, a second temperature sensor, a thermal insulation layer positioned intermediate the first and second temperature sensor and a heater for heating the apparatus and a subdermal tissue region underlying the user's skin.

US 2018/0014734 relates to tissue-mounted devices and techniques for monitoring a thermal transport property of tissue, such as skin. The devices conformally mount to the tissue and comprise one or more thermal actuators and a plurality of sensors. The actuator applies heat to the tissue and the sensors detect a spatio temporal distribution of a physiological tissue parameter or physical property resulting from the heating.

US 2012/0296224 A1 relates to determining or measuring a biological, physical or physiological parameter of an object by a sensor. A sensor is provided, e.g. a flow sensor, employing a degradable adhesive for attachment of the sensor to the object. The degradable adhesive may be degradable e.g. by time, by exposure to a certain measure, e.g. induced heat, or substance for detaching the sensor from the object for subsequent removal of the sensor.

SUMMARY OF THE INVENTION

The invention is claimed in the independent claims. Preferred embodiments are specified in the dependent claims.

SUMMARY OF THE DISCLOSURE

According to an aspect of the present disclosure, an electronic device may include: a sensor including a heat source, a pair of first temperature sensors horizontally spaced apart from each other at two opposing sides of the heat source, and a pair of second temperature sensors vertically spaced apart from the pair of first temperature sensors in a thickness direction of the electronic device; and at least one processor configured to: control the heat source to generate heat while the electronic device is in contact with a user; estimate a blood temperature difference between the two opposing sides of the heat source, based on temperatures measured by the pair of first temperature sensors and the pair of second temperature sensors; and estimate blood flow information of the user based on the blood temperature difference.

The at least one processor may be further configured to: calculate a first temperature difference between the temperatures measured by the pair of first temperature sensors and a second temperature difference between the temperatures measured by the pair of second temperature sensors; and estimate the blood temperature difference based on the first temperature difference and the second temperature difference.

The at least one processor may be further configured to: obtain an objective function that uses a relational expression between the first temperature difference and the blood temperature difference and a relational expression between the second temperature difference and the blood temperature difference; and obtain the blood temperature difference at which the objective function is minimized.

The sensor may further include a third temperature sensor horizontally spaced apart from the heat source, wherein the at least one processor may be further configured to correct the temperatures measured by the first temperature sensors based on temperature measured by the third temperature sensor.

The at least one processor is further configured to control an on-time period and an off-time period of the heat source according to a predetermined cycle, wherein the first temperature sensors and the second temperature sensors may be configured to measure temperatures during the off-time period of the heat source that follows the on-time period of the heat source.

The blood flow information may include at least one of a blood flow velocity, a blood flow direction, a core body temperature, and a blood glucose change.

The at least one processor may be further configured to estimate the blood flow velocity based on the blood temperature difference by using an estimation model that defines a correlation between the blood temperature difference and the blood flow velocity.

The at least one processor may be further configured to: estimate heat flux based on a difference between the temperatures measured by the first temperature sensor and the second temperature sensor which are disposed on at least one side of the heat source, and estimate the core body temperature based on the estimated heat flux.

Upon estimating the blood flow velocity, the at least one processor may be further configured to: calculate a difference between the estimated blood flow velocity and a reference blood flow velocity estimated at a reference time; and predict a change in blood glucose compared to the reference time based on the difference between the estimated blood flow velocity and a reference blood flow velocity.

At least one of the first temperature sensors and the second temperature sensors may be a thermistor.

The heat source has a diameter of 1 mm to 10 mm.

A distance between a center and the two opposing sides of the heat source is within 8.4 mm.

A distance between the pair of first temperature sensors and the pair of second temperature sensors is 0.4 mm to 10 mm.

The sensor may include a thermally insulating materials disposed between the first temperature sensors and between the second temperature sensors.

According to another aspect of the present disclosure, a method of estimating blood flow information by an electronic device, may include: controlling a heat source to generate heat while the electronic device is in contact with a user; measuring temperatures by using a pair of first temperature sensors horizontally spaced apart from each other at two opposing sides of the heat source, and a pair of second temperature sensors vertically spaced apart from the pair of first temperature sensors in a thickness direction of the electronic device; estimating a blood temperature difference between the two opposing sides of the heat source, based on the temperatures measured by the pair of first temperature sensors and the pair of second temperature sensors; and estimating blood flow information of the user based on the blood temperature difference.

The estimating of the blood temperature difference may include: calculating a first temperature difference between the temperatures measured by the pair of first temperature sensors and a second temperature difference between the temperatures measured by the pair of second temperature sensors; and estimating the blood temperature difference based on the first temperature difference and the second temperature difference.

The method may further include measuring a temperature by using a third temperature sensor horizontally spaced apart from the heat source, wherein the estimating of the blood temperature difference may include correcting the temperatures measured by the first temperature sensors based on the temperature measured by the third temperature sensor.

The controlling of the heat source may include: controlling an on-time period and an off-time period of the heat source according to a predetermined cycle, wherein the measuring of the temperatures may include measuring temperatures during the off-time period of the heat source that follows the on-time period of the heat source.

The estimating of the blood flow information may include estimating a blood flow velocity, included in the blood flow information, based on the estimated blood temperature difference by using an estimation model that defines a correlation between the blood temperature difference and the blood flow velocity.

According to another aspect of the present disclosure, a patch-type sensor may include: a contact pad configured to make contact with a skin surface; a heat source configured to apply heat to the skin surface when the contact pad makes contact with the skin surface; a pair of first temperature sensors horizontally spaced apart from each other at two opposing sides of the heat source; a pair of second temperature sensors vertically spaced apart from the pair of first temperature sensors in a depth direction of the patch-type sensor; and a communication interface configured to transmit temperature data, measured by the pair of first temperature sensors and the pair of the second temperature sensors, to an external electronic device, to enable the external electronic device to obtain blood flow information by estimating a blood temperature difference based on the temperature data.

BRIEF DESCRIPTION OF THE DRAWINGS

The above and/or other aspects will be more apparent by describing certain example embodiments, with reference to the accompanying drawings, in which:

FIG. 1 is a block diagram illustrating an example of an electronic device for estimating blood flow information;
FIG. 2 is a diagram illustrating an example of a sensor structure;
FIG. 3 is a diagram explaining operation of a heat source of a sensor;
FIG. 4 is a block diagram illustrating another example of an electronic device for estimating blood flow information;
FIG. 5 is a view, as seen from above, of a sensor being in contact with an object;
FIG. 6 is a block diagram illustrating yet another example of an electronic device for estimating blood flow information;
FIGS. 7A and 7B are diagrams illustrating examples of outputting blood flow information;
FIG. 8 is a block diagram illustrating yet another example of an electronic device for estimating blood flow information;
FIG. 9 is a flowchart illustrating a method of estimating blood flow information according to an embodiment of the present disclosure;
FIG. 10 is a flowchart illustrating a method of estimating blood flow information according to another embodiment of the present disclosure;
FIGS. 11 to 14 are diagrams illustrating examples of structures of an electronic device according to embodiments of the present disclosure; and
FIGS. 15 and 16 are diagrams illustrating examples of obtaining blood flow information by using two or more devices operating in conjunction with each other.

DETAILED DESCRIPTION

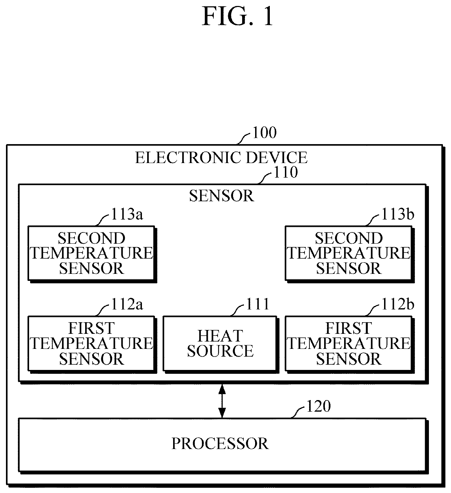

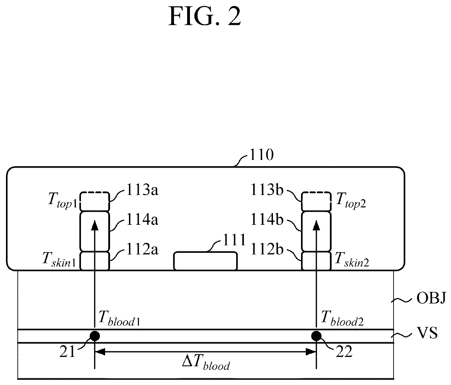

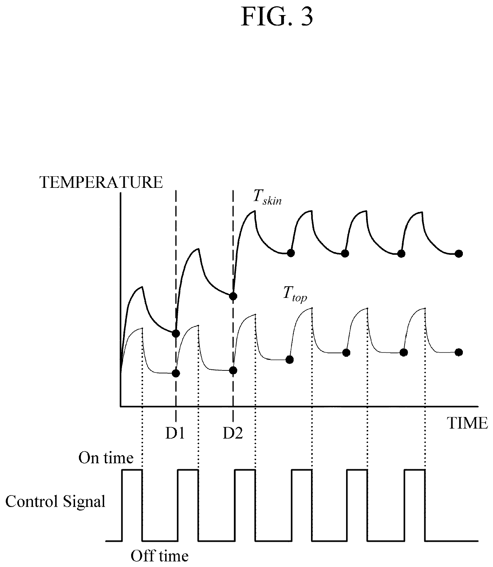

Example embodiments are described in greater detail below with reference to the accompanying drawings.
In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.
It will be understood that, although the terms "first," "second," etc. may be used herein to describe various elements, these elements should not be limited by these terms. These terms are only used to distinguish one element from another. Any references to singular may include plural unless expressly stated otherwise. In addition, unless explicitly described to the contrary, an expression such as "comprising" or "including" will be understood to imply the inclusion of stated elements but not the exclusion of any other elements. Also, the terms, such as 'unit' or 'module', etc., should be understood as a unit that performs at least one function or operation and that may be embodied as hardware, software, or a combination thereof.
Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.
An electronic device according to various embodiments of the present disclosure which will be described below may be, for example, a wearable device, a smartphone, a tablet PC, a mobile phone, a video phone, an electronic book reader, a desktop computer, a laptop computer, a netbook computer, a workstation, a server, a PDA, a portable multimedia player (PMP), an MP3 player, a medical device, and a camera. The wearable device may include at least one of an accessory type wearable device (e.g., wristwatch, ring, bracelet, anklet, necklace, glasses, contact lens, or head mounted device (HMD)), a textile/clothing type wearable device (e.g., electronic clothing), a body-mounted type wearable device (e.g., skin pad or tattoo), and a body implantable type wearable device. However, the wearable device is not limited thereto and may include, for example, various portable medical measuring devices (antioxidant measuring device, blood glucose monitor, heart rate monitor, blood pressure measuring device, thermometer, etc.), magnetic resonance angiography (MRA), magnetic resonance imaging (MRI), computed tomography (CT), imaging system, ultrasonic system, etc.), and the like. However, the electronic device is not limited to the above devices
FIG. 1 is a block diagram illustrating an electronic device according to an embodiment of the present disclosure. FIG. 2 is a diagram illustrating an example of a sensor structure. FIG. 3 is a diagram explaining operation of a heat source of a sensor.
Referring to FIGS. 1 and 2, an electronic device 100 includes a sensor 110 and a processor 120.
The sensor 110 may include a heat source 111 and a plurality of temperature sensors 112a, 112b, 113a, and 113b. The heat source 111 and the plurality of temperature sensors 112a, 112b, 113a, and 113b may be mounted on a sensor board. In this case, the sensor board may be, for example, a flexible printed circuit board (FPCB) having flexible characteristics and capable of being bent in a predetermined shape. For example, the sensor board may have a size of 4 cm x 2 cm, but is not limited thereto, and may be changed to various sizes according to the size of a form factor.

The heat source 111 may be disposed in the sensor 110 at a position adjacent to a contact position of an object OBJ, and may apply heat to the object OBJ during contact with the object OBJ. The heat source 111 may be made of a thin (and flat) metal film or wire, such as platinum, and may be formed by connecting one or more resistors. The heat source 111 may be connected to a power supply configured to provide power to the heat source 111 to generate heat, wherein the power may be current supplied from a General Purpose Input/Output (GPIO) of Bluetooth Low Energy SoC (BLE SoC), but examples of the power supply are not limited thereto.

The plurality of temperature sensors 112a, 112b, 113a, and 113b may be contact type temperature sensors (e.g., thermistors), but are not limited thereto, and some may be thermistors and the others may be non-contact type temperature sensors (e.g., infrared temperature sensors). A pair of first temperature sensors 112a and 112b may be horizontally spaced apart from each other at both sides of the heat source 111 so as to measure a surface temperature of the object OBJ. In addition, a pair of second temperature sensors 113a and 113b may be vertically spaced apart from the first temperature sensors 112a and 112b, respectively, so as to measure temperature at a position away from a surface of the object OBJ. However, the second temperature sensors 113a and 113b are not necessarily disposed in a line at positions vertically spaced apart from the first temperature sensors 112a and 112b in a thickness direction of the sensor 110, and the positions thereof are not limited thereto as long as the second temperature sensors 113a and 113b are disposed further away from the object OBJ than the first temperature sensors 112a and 112b.

The heat source 111 may have a diameter in a range of 1 mm to 10 mm. For example, in the case where the electronic device 100 is a smartwatch, the heat source 111 may have a diameter in a range of 1 mm to 5 mm, and the diameter may be, for example, 3 mm. In addition, in the case where the electronic device 100 is a patch-type device, the heat source 111 may have a diameter in a range of 1 mm to 10 mm, and the diameter may be, for example, 10 mm. The heat source 111 may have various shapes, such as a circular shape, a tetragonal shape, etc., with no particular limitation.

The first temperature sensors 112a and 112b, disposed at both sides of the heat source 111 in a horizontal direction, may be disposed within 8.4 mm from the center of the heat source 111. For example, in the case where the electronic device 100 is a smartwatch, each of the first temperature sensors 112a and 112b may be disposed at a distance of 4.2 mm $\pm$ 1 mm from the center of the heat source 111. In addition, in the case where the electronic device 100 is a patch-type device, each of the first temperature sensors 112a and 112b may be disposed at a distance of 8.2 mm from the center of the heat source 111. However, the distance is not limited thereto, and may vary according to the size of a form factor.

Further, the heat source 111 and the first temperature sensors 112a and 112b may be disposed at a distance of approximately 0.4 mm to 10 mm from the surface of the object OBJ during contact with the object OBJ. In addition, a distance between the first temperature sensors 112a and 112b and the second temperature sensors 113a and 113b in the thickness direction of the sensor 110 may be in a range of 0.4 mm to 10 mm. For example, in the case where the electronic device 100 is a smartwatch, the distance may range from 0.4 mm to 1.3 mm. However, the distance is not limited thereto, and may vary according to the size of a form factor.

As illustrated in FIG. 2, the sensor 110 may further include low thermal conductors 114a and 114b. The low thermal conductors 114a and 114b may be disposed between the first temperature sensors 112a and 112b and the second temperature sensors 113a and 113b and may maintain a constant distance between the first temperature sensors 112a and 112b and the second temperature sensors 113a and 113b. The low thermal conductor may be formed of, for example, an acrylic material, but is not limited thereto, and may be formed of, for example, insulation materials such as fiberglass, rock wool, and foam materials, plastics such as polystyrene and polyurethane, rubbers, elastomers, glass, air, certain ceramics, such as alumina and silica, and any other material with a thermal conductivity of 0.1 W/mK or less. The size or thermal conductivity of the low thermal conductor is not limited thereto. In addition, without using a separate thermally low conductive material, a structure filled with air having a relatively low thermal conductivity is also possible.

The processor 120 may be electrically connected to the sensor 110 via wire and/or wirelessly. The processor 120 may control the sensor 110 to obtain temperature data from the object OBJ. The object OBJ may be a surface of the wrist that is adjacent to the radial artery or an upper part of the wrist where venous blood or capillary blood passes, or a peripheral part of the body with high blood vessel density, such as fingers, toes, ears, or the like.

The processor 120 may receive temperature data from the sensor 110 and may estimate blood flow information by using the received temperature information. The blood flow information may include a variety of information related to vascular health, including a blood flow velocity, blood flow direction, core body temperature, blood glucose change, blood pressure change, arterial stiffness, vascular age, and the like.

In order to estimate the blood flow information, the processor 120 controls the heat source 111 to apply heat to the object OBJ. Once heat is applied to the object OBJ, a change in blood temperature is induced at two points 21 and 22 in a blood vessel VS of the object OBJ. In this case, the pair of first temperature sensors 112a and 112b at both sides of the heat source 111 measures first temperatures $T_{sink1}$ and $T_{sink2}$ on the surface of the object OBJ, and the pair of second temperature sensors 113a and 113b measures second temperatures $T_{top1}$ and $T_{top2}$ at positions relatively further away from the surface of the object OBJ than the first temperature sensors 112a and 112b.

In this case, when the second temperatures $T_{top1}$ and $T_{top2}$ are measured while the heat source 110 is still turned on, the heat source 110 may interfere with heat transfer from the first temperature sensors 112a and 112b toward the second temperature sensors 113a and 113b, thereby reducing measurement accuracy. Accordingly, the processor 120 may periodically turns on and off the heat source 111 as illustrated in FIG. 3 according to a control signal, and the first temperature sensors 112a and 112b and the second temperature sensors 113a and 113b measure the first temperatures $T_{sink1}$ and $T_{sink2}$ and the second temperatures $T_{top1}$ and $T_{top2}$ in a stable state at a specific point D1 or D2 immediately before the heat source 110 is turned on, while the heat source 110 is turned off. In other words, the first temperatures $T_{sink1}$ and $T_{sink2}$ and the second temperatures $T_{top1}$ and $T_{top2}$ are measured at the end of an off-time period, D1 or D2, which follows an on-time period.

The processor 120 may receive first temperature data and second temperature data measured by the first temperature sensors 112a and 112b and the second temperature sensors 113a and 113b, and may estimate blood flow information by using the received first temperature and second temperature. The processor 120 may estimate a blood temperature difference between the two points 21 and 22 of the blood vessel VS at both sides of the heat source 111, and may estimate a blood vessel velocity by using the blood temperature difference.

For example, the processor 120 may obtain a first temperature difference ($\Delta T_{skin} = T_{skin2} - T_{skin1}$) between the first temperatures $T_{sink1}$ and $T_{sink2}$ measured by the first temperature sensors 112a and 112b. In addition, the processor 120 may obtain a second temperature difference ($\Delta T_{top} = T_{top2} - T_{top1}$) between the second temperatures $T_{top1}$ and $T_{top2}$ measured by the second temperature sensors 113a and 113b. The temperatures $T_{top1}$, $T_{top2}$, $T_{sink1}$, and $T_{sink2}$ may be defined as shown in the following Equations 1 and 2, which may be rearranged to derive the following Equations 3 and 4 that define a relationship between the first temperature difference $\Delta T_{skin}$, the second temperature difference $\Delta T_{top}$, and blood temperature difference $\Delta T_{blood} = T_{blood2} - T_{blood1}$.

[Equation 1]

$$T_{skin1} = T_{room} + \frac{T_{blood1} - T_{room}}{\frac{t_{tissue}}{k_{tissue}} + \frac{t_{device}}{k_{device}} + \frac{1}{h}} \times \left( \frac{t_{device}}{k_{device}} + \frac{1}{h} \right)$$

$T_{sink2}$ may also be defined in a manner similar to $T_{sink1}$, as follows.

$$T_{skin2} = T_{room} + \frac{T_{blood2} - T_{room}}{\frac{t_{tissue}}{k_{tissue}} + \frac{t_{device}}{k_{device}} + \frac{1}{h}} \times \left( \frac{t_{device}}{k_{device}} + \frac{1}{h} \right)$$

[Equation 2]

$$T_{top1} = T_{room} + \frac{T_{blood1} - T_{room}}{\frac{t_{tissue}}{k_{tissue}} + \frac{t_{device}}{k_{device}} + \frac{1}{h}} \times \left( \frac{1}{h} \right)$$

$T_{top2}$ may also be defined in a manner similar to $T_{top1}$, as follows.

$$T_{top2} = T_{room} + \frac{T_{blood2} - T_{room}}{\frac{t_{tissue}}{k_{tissue}} + \frac{t_{device}}{k_{device}} + \frac{1}{h}} \times \left( \frac{1}{h} \right)$$

[Equation 3]

$$\Delta T_{skin} = \frac{\frac{t_{device}}{k_{device}} + \frac{1}{h}}{\frac{t_{tissue}}{k_{tissue}} + \frac{t_{device}}{k_{device}} + \frac{1}{h}} \times (\Delta T_{blood})$$

[Equation 4]

$$\Delta T_{top} = \frac{\frac{1}{h}}{\frac{t_{tissue}}{k_{tissue}} + \frac{t_{device}}{k_{device}} + \frac{1}{h}} \times (\Delta T_{blood})$$

In Equations 1 to 4, $T_{room}$ denotes ambient temperature around the sensor 110 and may denote, for example, temperature inside or outside the apparatus 100. $T_{room}$ is erased in the process of obtaining the first temperature difference $\Delta T_{skin}$ and the second temperature difference $\Delta T_{top}$, such that there is no need for a separate temperature sensor for measuring ambient temperature. Subscripts tissue and device denote the object OBJ and a sensor layer 110, $t_{device}$ and $k_{device}$ denote the thickness and thermal conductivity of the sensor 110, respectively, and may be previously input values, and $t_{tissue}/k_{tissue}$ denote tissue characteristics equivalent to the core body temperature of the object and $t_{tissue}$ and $k_{tissue}$ may denote the thickness and thermal conductivity, respectively, h denotes a heat transfer coefficient, and $\Delta T_{blood}$ denotes the blood temperature difference. The first temperatures $T_{sink1}$ and $T_{sink2}$ and the second temperatures $T_{top1}$ and $T_{top2}$ denote values measured by the first temperature sensors 112a and 112b and the second temperature sensors 113a and 113b, the first temperature difference $\Delta T_{skin}$ and the second temperature difference $\Delta T_{top}$ are values obtained based on the first temperatures $T_{sink1}$ and $T_{sink2}$ and the second temperatures $T_{top1}$ and $T_{top2}$, in which $t_{tissue}/k_{tissue}$, h, and $\Delta T_{blood}$ are unknowns.

By using Equations 3 and 4 that define a relationship between the first temperature difference $\Delta T_{skin}$, the second temperature difference $\Delta T_{top}$, and the blood temperature difference $\Delta T_{blood}$, the processor 120 may derive an objective function as shown in the following Equation 5.

[Equation 5]

$$\Pi = \left[ \frac{\Delta T_{blood}}{\Delta T_{top}} \frac{\frac{1}{h}}{\frac{t_{tissue}}{k_{tissue}} + \frac{t_{device}}{k_{device}} + \frac{1}{h}} - 1 \right]^2 + \left[ \frac{\Delta T_{blood}}{\Delta T_{skin}} \frac{\frac{t_{device}}{k_{device}} + \frac{1}{h}}{\frac{t_{tissue}}{k_{tissue}} + \frac{t_{device}}{k_{device}} + \frac{1}{h}} - 1 \right]^2$$

As described above, the processor 120 may obtain unknowns $t_{device}/k_{device}$, h, and $\Delta T_{blood}$ at which the derived objective function $\Pi$ is minimized. The objective function $\Pi$ may be stored in a storage of the electronic device 100, to obtain $t_{device}/k_{device}$, h, and $\Delta T_{blood}$ based on the objective function $\Pi$ and the temperatures measured by the sensor 110. The objective function $\Pi$ may be determined to be minimized when the output value of the objective function $\Pi$ reaches a local minimum value or a predetermined minimum value.

Upon obtaining the blood temperature difference $\Delta T_{blood}$ as described above, the processor 120 may estimate a blood flow velocity by using the obtained blood temperature difference. For example, the processor 120 may obtain the blood flow velocity by inputting the blood temperature difference to an estimation model that defines a correlation between the blood temperature difference and the blood flow velocity. In this case, the estimation model may be a linear regression equation or a neural network-based model trained by deep learning.

In the above Equation 3, t, k, and h are all positive numbers, such that a relationship of $\Delta T_{blood} > \Delta T_{skin}$ is satisfied. In the following Equation 6, t, k, and h are irrelevant to a blood flow velocity f, such that a relationship of $\frac{d\Delta T_{blood}}{df} > \frac{d\Delta T_{skin}}{df}$ is satisfied, and it can be seen that the blood temperature difference $\Delta T_{blood}$ is more sensitive to a small flow velocity variation $\Delta f$ than the first temperature difference $\Delta T_{skin}$.

[Equation 6]

$$\frac{d\Delta T_{blood}}{df} = \frac{\frac{t_{tissue}}{k_{tissue}} + \frac{t_{device}}{k_{device}} + \frac{1}{h}}{\frac{t_{device}}{k_{device}} + \frac{1}{h}} \times \frac{d\Delta T_{skin}}{df}$$

In a conventional thermal mass flowmeter, only a temperature sensor on the skin surface is used, leading to a small temperature difference before and after the heat source, such that the accuracy of a blood flow velocity is reduced. As described above, in this embodiment, the blood temperature difference is estimated using the second temperature sensors 113a and 113b vertically disposed over the first temperature sensors 112a and 112b, and the blood flow velocity is estimated based on the blood temperature difference, thereby obtaining a high resolution compared to the case where only the temperature difference on the skin surface is used. In addition, as can be seen from the above Equation, there is no need for a separate sensor for measuring ambient temperature, thereby preventing an increase in device volume.

Further, the processor 120 may estimate a core body temperature by using the first temperature $T_{skin1}$ and the second temperature $T_{top1}$ which are measured by the first temperature sensor 112a and the second temperature sensor 113a disposed on one side of the heat source 111. For example, assuming that a flow of heat is current, a heat transfer characteristic of a material is resistance, and heat flux is voltage, the heat flow may be expressed in equation form according to Ohm's law (V=IR), and a temperature difference ($T_{skin1}$ - $T_{top1}$) in the material may be estimated as the heat flux Q. Then, by combining the estimated heat flux Q with the surface temperature $T_{skin1}$ of a body part to be measured, the processor 120 may estimate a core temperature at a body measurement position. Based on the relationship, Equation 7 for estimating the core body temperature at the body measurement position may be derived.

[Equation 7]

$$T_{core} = T_{skin1} + \frac{(T_{skin1} - T_{top1})}{\varepsilon}$$

Herein, $\varepsilon$ denotes a predetermined coefficient, $T_{core}$ denotes the core body temperature, $T_{skin1}$ denotes the temperature measured by the first temperature sensor 112a, $T_{top1}$ denotes the temperature measured by the second temperature sensor 113a, and $T_{skin1}$ - $T_{top1}$ denotes the heat flux Q.

In another example, the processor 120 may estimate a core body temperature by inputting the first temperature, the heat flux, and the obtained blood flow velocity to a predefined core body temperature estimation model.

Likewise, the processor 120 may estimate a core body temperature by using the first temperature $T_{skin2}$ and the second temperature $T_{top2}$ measured by the first temperature sensor 112b and the second temperature sensor 113b which are disposed on the other side of the heat source 111, or the processor 120 may obtain, as the core body temperature, a statistical value (e.g., mean value) of core body temperature values measured by using all the temperature sensors at both sides of the heat source 111.

In addition, the processor 120 may estimate a blood flow direction. For example, the processor 120 may estimate the blood flow direction based on a positive or negative sign of the first temperature difference ($\Delta T_{skin} = T_{skin2} - T_{skin1}$). It can be estimated that if the first temperature difference $\Delta T_{skin}$ is positive, the blood flows from the point 21 toward the point 22, and if the first temperature difference $\Delta T_{skin}$ is negative, the blood flows from the point 22 toward the point 21.

Further, upon estimating the blood flow velocity, the processor 120 may obtain a variation in blood flow velocity by comparing the estimated blood flow velocity with a blood flow velocity estimated at a reference time, and may estimate information, such as arterial stiffness, blood glucose variation, vascular age, etc., based on the variation in blood flow velocity. For example, if the variation in blood flow velocity is less than or equal to a threshold value, the processor 120 may predict an increase in blood glucose levels, leading to an increase in blood viscosity. Further, if the variation in blood flow velocity is less than or equal to the threshold value, the processor 120 may predict an increase in arterial stiffness. As described above, upon predicting the increase in blood glucose levels and/or arterial stiffness based on the variation in blood flow velocity, the processor 120 may generate and provide warning information for a user.

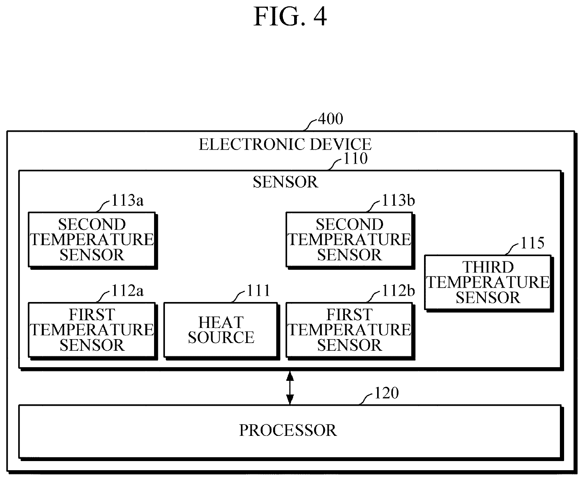

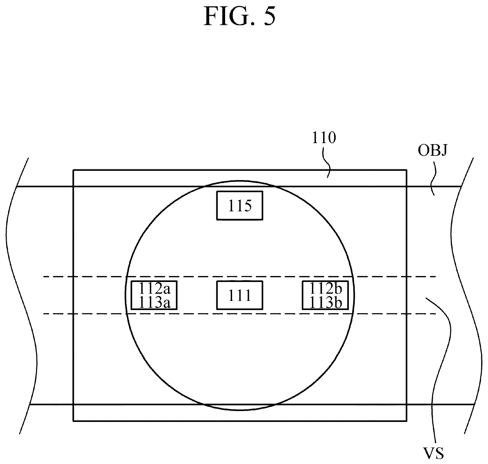

FIG. 4 is a block diagram illustrating another example of an electronic device for estimating blood flow information. FIG. 5 is a view, as seen from above, of a sensor being in contact with an object.

Referring to FIG. 4, an electronic device 400 includes the sensor 110 and the processor 120. The sensor 110 may include the heat source 111, the first temperature sensors 112a and 112b, the second temperature sensors 113a and 113b, and the third temperature sensor 115. Further, the electronic device 400 may further include low thermal conductors disposed between the first temperature sensors 112a and 112b and the second temperature sensors 113a and 113b as described above.

The heat source 111 and the pair of first temperature sensors 112a and 112b may be arranged side by side at a contact position with the object. The pair of second temperature sensors 113a and 113b may be vertically disposed over the pair of first temperature sensors 112a and 112b at a position further away from the object.

As illustrated in FIG. 5, when the sensor 110 or the electronic device 100 including the sensor 110 makes contact with the object OBJ, the heat source 111, the pair of first temperature sensors 112a and 112b, and the pair of second temperature sensors 113a and 113b may be in contact at a position where the blood vessel VS passes, and the third temperature sensor 115 may be in contact at a position where the blood vessel VS does not pass.

As described above, when a blood temperature difference is induced by operating the heat source 111, the first temperature sensors 112a and 112b and the second temperature sensors 113a and 113b may measure first temperatures on the surface at two points where the blood vessel VS of the object OBJ passes and measure second temperatures of a space away from the surface. The third temperature sensor 115 may measure a third temperature on the surface of the object where the blood vessel does not pass. The temperature sensors 112a, 112b, 113a, and 113b may be thermistors.

The processor 120 may correct the first temperatures on the skin surface, which are measured by the first temperature sensors 112a and 112b, by using the third temperature measured by the third temperature sensor 115. For example, the processor 120 may obtain the corrected first temperatures by subtracting the third temperature from each of the first temperatures, but is not limited thereto. The processor 120 may estimate a blood temperature difference based on the corrected first temperatures and the second temperatures as described above, and may estimate the blood flow velocity by using the estimated blood temperature difference. In addition, the processor 120 may monitor a variety of cardiovascular information, such as the core body temperature, blood glucose change, arterial stiffness, vascular age, etc., and may provide a user with warning information and the like.

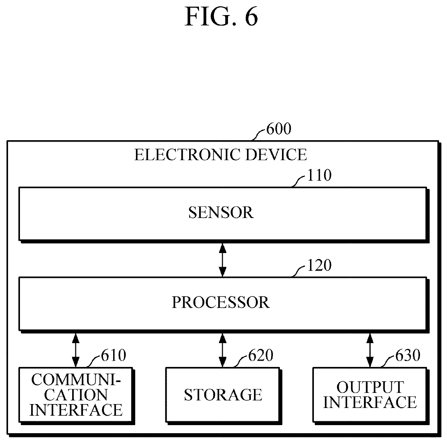

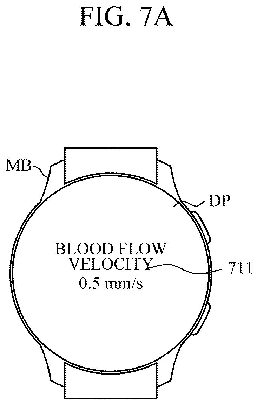

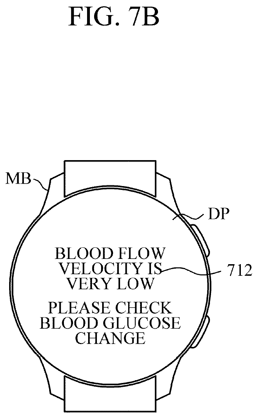

FIG. 6 is a block diagram illustrating yet another example of an electronic device for estimating blood flow information. FIGS. 7A and 7B are diagrams illustrating examples of outputting blood flow information.

Referring to FIG. 6, an electronic device 600 may further include a communication interface 610, a storage 620, and an output interface 630, in addition to the sensor 110 and the processor 120.

The sensor 110 includes the heat source 111, the first temperature sensors 112a and 112b, the second temperature sensors 113a and 113b, and/or the third temperature sensor 115, as illustrated in FIG. 1 or FIG. 4.

The processor 120 may control the heat source 111, may estimate a blood temperature difference by using the temperatures measured by the plurality of temperature sensors, and may estimate blood flow information, such as a blood flow velocity and the like, by using the estimated blood temperature difference.

The communication interface 610 may transmit temperature data, measured by the plurality of temperature sensors, to another electronic device. Further, the communication interface 610 may transmit the blood flow information, such as the blood temperature difference, blood flow velocity, etc., which are estimated by the processor 120, to another electronic device. In addition, the communication interface 610 may receive temperature data measured by another electronic device or sensor.

The communication interface 610 may communicate with another external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

The storage 620 may store various instructions. In addition, the storage 620 may store various data necessary for estimating blood flow information, including a blood flow velocity estimation model, a heat transfer coefficient, a core body temperature estimation model, criteria for controlling a heat source, and the like. Further, the storage 620 may store user characteristic information, such as a user's age, height, weight, exercise information, health information, and the like. Moreover, the storage 620 may store various data processed and generated by the electronic device 600.

The storage 620 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The output interface 630 may output data processed by the electronic device 600, for example, information including the first temperature, second temperature, blood temperature difference, blood flow velocity, blood flow direction, core body temperature, blood glucose change, arterial stiffness, etc., or warning information in a visual/non-visual manner by using a display, an audio output module, a haptic module, and the like. For example, as illustrated in FIG. 7A, the output interface 630 may output the blood flow velocity information estimated by the processor 120. In this case, depending on whether the blood flow velocity is normal, the output interface 630 may output the blood flow velocity information in different colors, and may further output warning information as illustrated in FIG. 7B.

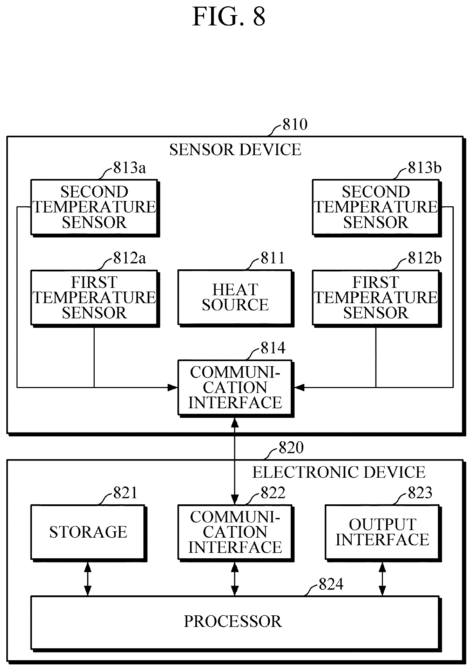

FIG. 8 is a block diagram illustrating yet another example of an electronic device for estimating blood flow information. Referring to FIG. 8, the electronic device for estimating blood flow information is formed as two or more separate devices 810 and 820 which may interwork with each other to estimate blood flow information.

The sensor device 810 is a device for measuring temperature data from an object and may include a heat source 811, first temperature sensors 812a and 812b, second temperature sensors 813a and 813b, and a communication interface 814. In addition, the sensor device 810 may further include a third temperature sensor. The sensor device 810 may be, for example, a patch-type sensor which comes into contact with an object. However, the sensor device 810 is not limited thereto, and may include various electronic device, such as a smartwatch, an ear-wearable device, a smartphone, and the like.

The communication interface 814 may include a BLE communication protocol, but is not limited thereto. The communication interface 814 may be connected to the heat source 811 and may apply, for example, a current from a GPIO of BLE SoC, to the heat source 811. The communication interface 814 may receive temperature data from the temperature sensors 812a, 812b, 813a, and 813b, and may transmit the receive temperature data to the electronic device 820. In this case, the communication interface 814 may convert the received temperature data into digital information through a Variable Gain Amplifier (VGA) and an Analog-to-Digital Converter (ADC), and may transmit the digital information to the electronic device 820.

The electronic device 820 may include a storage 821, a communication interface 822, an output interface 823, and a processor 824. The electronic device 820 may be a smartphone, a smartwatch, a table PC, a desktop computer, a medical institution server, etc., and is not particularly limited.

The communication interface 822 may receive temperature data from the sensor device 810 by using various communication techniques described above, and may transmit the temperature data to the processor 824. In addition, the communication interface 822 may transmit, to the sensor device 810, a signal for controlling the sensor device 810 under the control of the processor 824.

The processor 824 may estimate a blood temperature difference and blood flow information based on the received temperature data, as described above. The electronic device 820 may further include the sensor 110 of the electronic device 100 of FIG. 1 or FIG. 4. In this case, the processor 824 may control the sensor device 810 to obtain temperature data at a first position (e.g., in the vicinity of the upper arm) of an object and may obtain temperature data at a second position (e.g., wrist) of the object by using a sensor mounted therein, and may obtain respective blood flow values by using each of the temperature data measured at the first position and the temperature data measured at the second position, as described above. Further, the processor 824 may compare the obtained respective blood flow values or may calculate a statistical value (e.g., mean value) of the respective blood flow values.

The storage 821 may store temperature data, blood temperature difference, and/or blood flow information, etc., and the output interface 823 may output information generated by the processor 824, for example, the blood temperature difference, blood flow information, warning information, and the like.

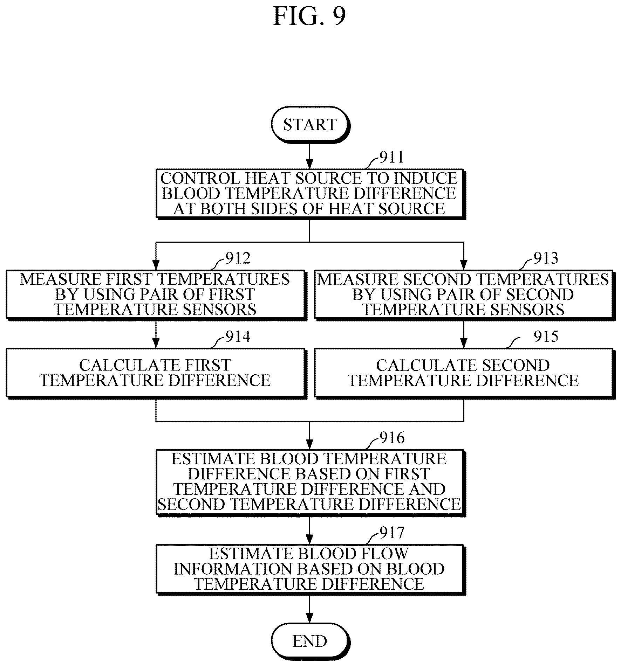

FIG. 9 is a flowchart illustrating a method of estimating blood flow information according to an embodiment of the present disclosure. The method of FIG. 9 may be performed by the electronic device of FIG. 1 or FIG. 6, which is described in detail above, and thus will be briefly described below in order to avoid redundancy.

First, the electronic device may control the heat source of the sensor in operation 911. The heat source may be disposed in the sensor at a position adjacent to a contact position of an object. The electronic device may periodically control the heat source, and by operating the heat source, the electronic device may induce a blood temperature difference between two points of the blood vessel at both sides of the heat source.

Then, the electronic device may measure first temperatures on the surface of the object by using the pair of first temperature sensors disposed side by side in a horizontal direction of the heat source in the sensor in operation 912. In addition, the electronic device may measure second temperatures at a position further away from the surface of the object by using the pair of second temperature sensors vertically spaced apart from the pair of first temperature sensors in the sensor in operation 913.

Subsequently, the electronic device may calculate, in operation 914, a first temperature difference between the first temperatures measured by the pair of first temperature sensors in operation 912. In addition, the electronic device may calculate, in operation 915, a second temperature difference between the second temperatures measured by the pair of second temperature sensors in operation 913.

Next, the electronic device may estimate a blood temperature difference based on the first temperature difference and the second temperature difference in operation 916. There is a relationship between the first and second temperature differences and the blood temperature difference as shown in Equations 3 and 4, and the electronic device may derive the objective function shown in Equation 5 based on the relationship, and may obtain a blood temperature difference at which the objective function is minimized.

Then, the electronic device may estimate blood flow information based on the blood temperature difference in operation 917. For example, the electronic device may estimate a blood flow velocity based on the blood temperature difference by using a blood flow velocity estimation model which defines a correlation between the blood temperature difference and the blood flow velocity. Further, as described above, the electronic device may obtain information, such as blood flow direction, core body temperature, and the like. Based on a relationship between a change in blood flow velocity and a change in blood glucose and arterial stiffness, the electronic device may monitor the change in blood glucose and arterial stiffness according to the change in blood flow velocity, and may generate warning information.

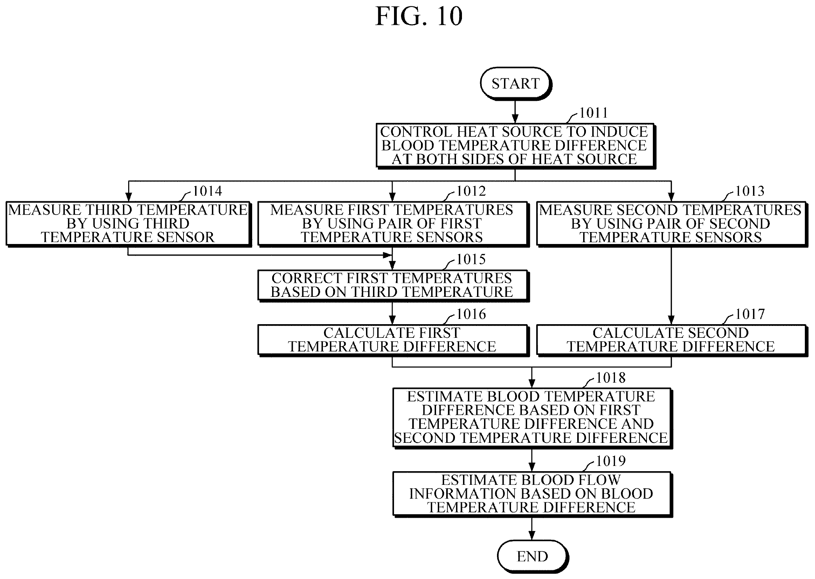

FIG. 10 is a flowchart illustrating a method of estimating blood flow information according to another embodiment of the present disclosure. The method of FIG. 10 may be performed by the electronic device of FIG. 4 or FIG. 6, which is described in detail above, and thus will be briefly described below in order to avoid redundancy.

First, the electronic device may control the heat source of the sensor to induce a blood temperature difference at both sides of the heat source in operation 1011. The electronic device may periodically control the heat source.

Then, the electronic device may measure first temperatures on the surface of the object by using the pair of first temperature sensors disposed side by side in a horizontal direction of the heat source in the sensor in operation 1012. In addition, the electronic device may measure second temperatures at a position further away from the surface of the object by using the pair of second temperature sensors vertically spaced apart from the pair of first temperature sensors in the sensor in operation 1013. Further, by using a third temperature sensor, the electronic device may measure a third temperature on the surface of the object at a position where the blood vessel of the object does not pass in operation 1014.

Subsequently, the electronic device may correct the first temperatures based on the third temperature in operation 1015. For example, the electronic device may obtain the corrected first temperatures by subtracting the third temperature from each of the first temperatures.

Next, the electronic device may calculate, in operation 1016, a first temperature difference between the first temperatures corrected in operation 1015. In addition, the electronic device may calculate, in operation 1017, a second temperature difference between the second temperatures measured by the pair of second temperature sensors in operation 1013.

Then, the electronic device may estimate a blood temperature difference based on the first temperature difference and the second temperature difference in operation 1018. There is a relationship between the first and second temperature differences and the blood temperature difference as shown in Equations 3 and 4, and the electronic device may derive the objective function shown in Equation 5 based on the relationship, and may obtain a blood temperature difference at which the objective function is minimized.

Subsequently, the electronic device may estimate blood flow information based on the blood temperature difference in 1019. For example, the electronic device may estimate a blood flow velocity based on the blood temperature difference by using a blood flow velocity estimation model which defines a correlation between the blood temperature difference and the blood flow velocity. Further, as described above, the electronic device may obtain information, such as blood flow direction, core body temperature, and the like. Based on a relationship between a change in blood flow velocity and a change in blood glucose and arterial stiffness, the electronic device may monitor the change in blood glucose and arterial stiffness according to the change in blood flow velocity, and may generate warning information.

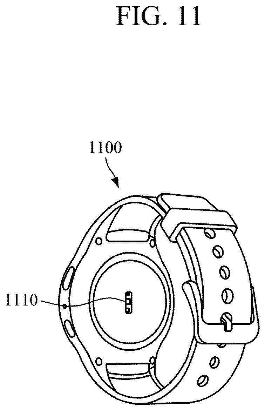

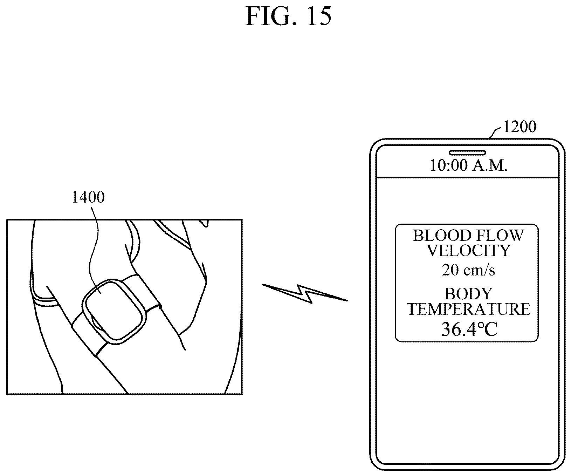

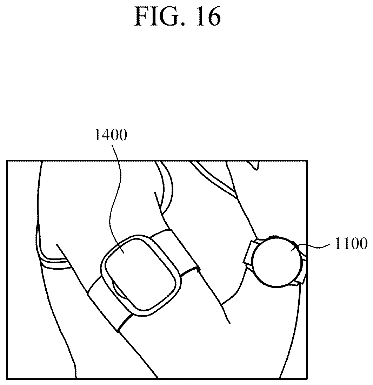

FIGS. 11 to 14 are diagrams illustrating examples of structures of an electronic device described above. FIGS. 15 and 16 are diagrams illustrating examples of obtaining blood flow information by using two or more devices operating in conjunction with each other.

The electronic device may include a sensor device, a processor, an input device, a communication module, a camera module, an output device, a storage device, and a power module. All the components of the electronic device may be integrally mounted in a specific device or may be distributed in two or more devices. The sensor device may include the heat source and the plurality of temperature sensors described above, and may further include an additional sensor, such as a gyro sensor, a Global Positioning System (GPS), and the like.

The processor may execute programs, stored in the storage device, to control components connected to the processor, and may perform various data processing or computation. For example, the processor may estimate blood flow information, including a blood temperature difference and blood flow velocity, based on temperature data measured on a surface of an object and temperature data measured at a position away from the object by using the plurality of temperature sensors of the sensor device. The processor may include a main processor, e.g., a central processing unit (CPU) or an application processor (AP), etc., and an auxiliary processor, e.g., a graphics processing unit (GPU), an image signal processor (ISP), a sensor hub processor, or a communication processor (CP), etc., which is operable independently from, or in conjunction with, the main processor.

The input device may receive a command and/or data to be used by each component of the electronic device, from a user and the like. The input device may include, for example, a microphone, a mouse, a keyboard, or a digital pen (e.g., a stylus pen, etc.).

The communication module may support establishment of a direct (e.g., wired) communication channel and/or a wireless communication channel between the electronic device and other electronic device, a server, or the sensor device within a network environment, and performing of communication via the established communication channel. The communication module may include one or more communication processors that are operable independently from the processor and supports a direct communication and/or a wireless communication. The communication module may include a wireless communication module, e.g., a cellular communication module, a short-range wireless communication module, or a global navigation satellite system (GNSS) communication module, etc., and/or a wired communication module, e.g., a local area network (LAN) communication module, a power line communication (PLC) module, and the like. These various types of communication modules may be integrated into a single chip, or may be separately implemented as multiple chips. The wireless communication module may identify and authenticate the electronic device in a communication network by using subscriber information (e.g., international mobile subscriber identity (IMSI), etc.) stored in a subscriber identification module.

The camera module may capture still images or moving images. The camera module may include a lens assembly having one or more lenses, image sensors, image signal processors, and/or flashes. The lens assembly included in the camera module may collect light emanating from a subject to be imaged.

The output device may visually/non-visually output data generated or processed by the electronic device. The output device may include a sound output device, a display device, an audio module, and/or a haptic module.

The sound output device may output sound signals to the outside of the electronic device. The sound output device may include a speaker and/or a receiver. The speaker may be used for general purposes, such as playing multimedia or playing record, and the receiver may be used for incoming calls. The receiver may be implemented separately from, or as part of, the speaker.

The display device may visually provide information to the outside of the electronic device. The display device may include, for example, a display, a hologram device, or a projector and control circuitry to control the devices. The display device may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may obtain the sound via the input device, or may output the sound via the sound output device, and/or a speaker and/or a headphone of another electronic device directly or wirelessly connected to the electronic device.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The storage device may store operating conditions required for operating the sensor device, and various data necessary for other components of the electronic device. The various data may include, for example, input data and/or output data for software and instructions associated with the software, and the like. The storage device may include a volatile memory and/or a non-volatile memory.

The power module may control power supplied to the electronic device. The power module may be implemented as part of, for example, a power management integrated circuit (PMIC). The power module may include a battery, which may include a primary cell which is not rechargeable, a secondary cell which is rechargeable, and/or a fuel cell.

Referring to FIG. 11, the electronic device may be implemented as a wristwatch wearable device 1100, and may include a main body and a wrist strap. A display is provided on a front surface of the main body, and may display various application screens, including a blood temperature difference, blood flow information, warning information, time information, received message information, and the like. A sensor device 1110 may be disposed on a rear surface of the main body. A processor and various other components may be disposed in the main body. A heat source and a plurality of temperature sensors may be disposed in the sensor device 1110.

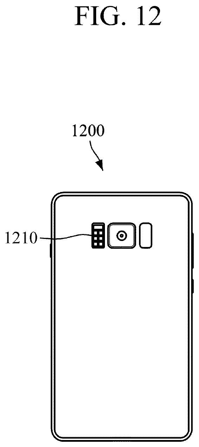

Referring to FIG. 12, the electronic device may be implemented as a mobile device 1200 such as a smartphone. The mobile device 1200 may include a housing and a display panel. The housing may form an exterior of the mobile device 1200. The housing has a first surface, on which a display panel and a cover glass may be disposed sequentially, and the display panel may be exposed to the outside through the cover glass. A sensor device 1210, a camera module and/or an infrared sensor, and the like may be disposed on a second surface of the housing. A processor and various other components may be disposed in the housing. A heat source and a plurality of temperature sensors may be disposed in the sensor device 1210.

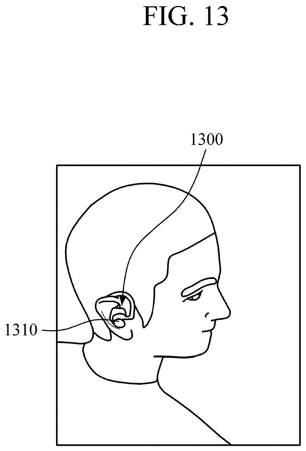

Referring to FIG. 13, the electronic device may be implemented as an ear-wearable device 1300. The ear-wearable device 1300 may include a main body and an ear strap. A user may wear the ear-wearable device 1300 by hanging the ear strap on the auricle. The ear strap may be omitted depending on a shape of the ear-wearable device 1300. The main body may be inserted into the external auditory meatus. A sensor device 1310 may be mounted in the main body at a contact portion with skin. The sensor device 1310 may include a heat source and a plurality of temperature sensors. Further, a processor, a communication device, and the like may be disposed in the main body.

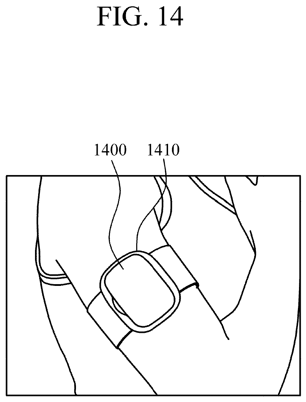

Referring to FIG. 14, an electronic device may be implemented as a patch-type wearable device 1400. The patch-type wearable device 1400 may have a contact pad to be attached to a surface (e.g., upper arm, wrist, etc.) of the object. In addition, a sensor board may be disposed therein, and a heat source and a plurality of temperature sensors may be mounted on the sensor board. Further, a processor, a communication device, and the like may be mounted in the patch-type wearable device 1400. The processor may obtain blood flow information based on temperature data measured by the plurality of temperature sensors.

FIG. 15 is a diagram illustrating an example of estimating blood flow information by using the patch-type wearable device 1400 and the mobile device 1200. As illustrated herein, when the patch-type wearable device 1400 is attached to the upper arm of the object and the sensor measures temperature in the vicinity of the upper arm, the patch-type wearable device 1400 may transmit the measured temperature data to the mobile device 1200. The mobile device 1200 may estimate blood flow information, e.g., blood flow velocity and/or core body temperature, by using the received temperature data, and may output an estimation result to a display. In this case, the patch-type wearable device 1400 may not include an algorithm for estimating the blood flow information.

FIG. 16 is a diagram illustrating an example of estimating blood flow information by using the patch-type wearable device 1400 and the smartwatch 1100. As illustrated herein, when the patch-type wearable device 1400 is attached to the upper arm of the object and the sensor measures temperature in the vicinity of the upper arm, the patch-type wearable device 1400 may transmit the measured temperature data to the smartwatch 1100. The smartwatch 1100 may estimate blood flow information in the vicinity of the upper arm by using the received temperature data. In this case, the patch-type wearable device 1400 may not include an algorithm for estimating the blood flow information. In addition, the smartwatch 1100 may measure temperature data from the wrist by using the sensor device 1110 mounted therein, and may estimate blood flow information in the vicinity of the wrist by using the measured temperature data. As described above, by simultaneously estimating the blood flow information in the vicinity of the upper arm and the wrist, estimation results at two positions, comparison data of the estimation results at two positions, or data obtained by integrating the estimation results at two positions, and the like may be provided to a user.

Aspects of the present disclosure can be realized as a computer-readable code written on a computer-readable recording/storage medium. The computer-readable recording/storage medium may be any type of recording device in which data is stored in a computer-readable manner.

Examples of the computer-readable recording/storage medium include a ROM, a RAM, a CD-ROM, a magnetic tape, a floppy disc, an optical data storage, and a carrier wave (e.g., data transmission through the Internet). The computer-readable recording/storage medium can be distributed over a plurality of computer systems connected to a network so that a computer-readable code is written thereto and executed therefrom in a decentralized manner. Functional programs, codes, and code segments needed for realizing the present invention can be readily deduced by programmers of ordinary skill in the art to which the invention pertains.

The present disclosure has been described herein with regard to preferred embodiments. However, it will be obvious to those skilled in the art that various changes and modifications can be made without changing technical conception and essential features of the present disclosure. Thus, it is clear that the above-described embodiments are illustrative in all aspects and are not intended to limit the present disclosure.

**Claims**

1. An electronic device (100) comprising:

    a sensor (110) that comprises a heat source (111), a pair of first temperature sensors (112a, 112b) horizontally spaced apart from each other at two opposing sides of the heat source, and a pair of second temperature sensors (113a, 113b) vertically spaced apart from the pair of first temperature sensors in a thickness direction of the electronic device; and
    at least one processor (120) configured to:

        control the heat source to generate heat while the electronic device is in contact with a user, wherein the controlling of the heat source comprises controlling an on-time period and an off-time period of the heat source according to a predetermined cycle; and
        receive first temperature data measured by the pair of first temperature sensors and second temperature data measured by the pair of second temperature sensors for enabling estimation of a blood temperature difference between the two opposing sides of the heat source, based on the first temperature data and the second temperature data, and for enabling estimation of blood flow information of the user based on the blood temperature difference,
        wherein the first and second temperature data is measured during the off-time period of the heat source that follows the on-time period of the heat source.

2. The electronic device claim 1, wherein the at least one processor is further configured to:
    calculate a first temperature difference between the temperatures measured by the pair of first temperature sensors and a second temperature difference between the temperatures measured by the pair of second temperature sensors for enabling estimation of the blood temperature difference based on the first temperature difference and the second temperature difference.

3. The electronic device of claim 2, wherein the at least one processor is further configured to:

    obtain an objective function that uses a relational expression between the first temperature difference and the blood temperature difference and a relational expression between the second temperature difference and the blood temperature difference; and
    obtain the blood temperature difference at which the objective function is minimized.

4. The electronic device of one of claims 1 to 3, wherein the sensor further comprises a third temperature sensor horizontally spaced apart from the heat source, and the at least one processor is further configured to correct the temperatures measured by the first temperature sensors based on temperature measured by the third temperature sensor.

5. The electronic device of one of claims 1 to 4, wherein the blood flow information comprises at least one of a blood flow velocity, a blood flow direction, a core body temperature, and a blood glucose change.

6. The electronic device of claim 5, wherein the at least one processor is further configured to enable one or more of:

estimating the blood flow velocity based on the blood temperature difference by using an estimation model that defines a correlation between the blood temperature difference and the blood flow velocity,
estimating heat flux based on a difference between the temperatures measured by the first temperature sensor and the second temperature sensor which are disposed on at least one side of the heat source, and
estimating the core body temperature based on the estimated heat flux.

7. The electronic device of claim 5, wherein upon estimating the blood flow velocity, the at least one processor further enables:

calculation of a difference between the estimated blood flow velocity and a reference blood flow velocity estimated at a reference time; and
prediction of a change in blood glucose compared to the reference time based on the difference between the estimated blood flow velocity and a reference blood flow velocity.

8. The electronic device of claim 1, wherein at least one of the first temperature sensors and the second temperature sensors is a thermistor, or

wherein the heat source has a diameter of 1 mm to 10 mm, or
wherein a distance between a center and the two opposing sides of the heat source is within 8.4 mm, or
wherein a distance between the pair of first temperature sensors and the pair of second temperature sensors is 0.4 mm to 10 mm, or
further comprising a thermally insulating materials disposed between the first temperature sensors and between the second temperature sensors.

9. A method of enabling estimation of blood flow information by an electronic device, the method comprising:

controlling (911; 1011) a heat source to generate heat while the electronic device is in contact with a user, wherein the controlling of the heat source comprises controlling an on-time period and an off-time period of the heat source according to a predetermined cycle;
measuring (912; 1012) first temperature data by using a pair of first temperature sensors horizontally spaced apart from each other at two opposing sides of the heat source, and measuring (913; 1013) second temperature data by using a pair of second temperature sensors vertically spaced apart from the pair of first temperature sensors in a thickness direction of the electronic device for enabling estimation of a blood temperature difference between the two opposing sides of the heat source, based on the first temperature data and the second temperature data, and for enabling estimation of blood flow information of the user based on the blood temperature difference,
wherein the first and second temperature data is measured during the off-time period of the heat source that follows the on-time period of the heat source.

10. The method of claim 9, further comprising:
calculating (914, 915; 1016, 1017) a first temperature difference between the temperatures measured by the pair of first temperature sensors and a second temperature difference between the temperatures measured by the pair of second temperature sensors for enabling estimation of the blood temperature difference based on the first temperature difference and the second temperature difference.

11. The method of claim 9 or 10, further comprising measuring (1014) a temperature by using a third temperature sensor horizontally spaced apart from the heat source,
wherein the estimating of the blood temperature difference comprises correcting the temperatures measured by the first temperature sensors based on the temperature measured by the third temperature sensor.

12. The method of one of claims 9 to 11, wherein enabling estimation of the blood flow information comprises enabling estimation of a blood flow velocity, included in the blood flow information, based on the estimated blood temperature difference by using an estimation model that defines a correlation between the blood temperature difference and the blood flow velocity.

**Patentansprüche**

1. Elektronische Vorrichtung (100), umfassend:

   einen Sensor (110), der eine Wärmequelle (111), ein Paar erster Temperatursensoren (112a, 112b), horizontal voneinander beabstandet an zwei gegenüberliegenden Seiten der Wärmequelle, und ein Paar zweiter Temperatursensoren (113a, 113b), vertikal beabstandet vom Paar erster Temperatursensoren in einer Dickenrichtung der elektronischen Vorrichtung, umfasst; und
   wenigstens einen Prozessor (120), ausgebildet zum:

   Steuern der Wärmequelle zum Erzeugen von Wärme, während die elektronische Vorrichtung in Kontakt mit einem Benutzer ist, wobei das Steuern der Wärmequelle das Steuern eines Einschaltzeitraums und eines Ausschaltzeitraums der Wärmequelle gemäß einem vorbestimmten Zyklus umfasst; und
   Empfangen erster Temperaturdaten, gemessen vom Paar erster Temperatursensoren, und zweiter Temperaturdaten, gemessen vom Paar zweiter Temperatursensoren, um das Schätzen eines Bluttemperaturunterschieds zwischen den beiden gegenüberliegenden Seiten der Wärmequelle basierend auf den ersten Temperaturdaten und den zweiten Temperaturdaten zu ermöglichen und um das Schätzen von Blutflussinformationen des Benutzers basierend auf dem Bluttemperaturunterschied zu ermöglichen,
   wobei die ersten und zweiten Temperaturdaten während des Ausschaltzeitraums der Wärmequelle gemessen werden, der auf den Einschaltzeitraum der Wärmequelle folgt.

2. Elektronische Vorrichtung nach Anspruch 1, wobei der wenigstens eine Prozessor ferner ausgebildet ist zum: Berechnen eines ersten Temperaturunterschieds zwischen den vom Paar erster Temperatursensoren gemessenen Temperaturen und eines zweiten Temperaturunterschieds zwischen den vom Paar zweiter Temperatursensoren gemessenen Temperaturen, um das Schätzen des Bluttemperaturunterschieds basierend auf dem ersten Temperaturunterschied und dem zweiten Temperaturunterschied zu ermöglichen.

3. Elektronische Vorrichtung nach Anspruch 2, wobei der wenigstens eine Prozessor ferner ausgebildet ist zum: Ermitteln einer Zielfunktion, die einen Vergleichsausdruck zwischen dem ersten Temperaturunterschied und dem Bluttemperaturunterschied und einen Vergleichsausdruck zwischen dem zweiten Temperaturunterschied und dem Bluttemperaturunterschied verwendet; und Ermitteln des Bluttemperaturunterschieds, bei dem die Zielfunktion minimiert wird.

4. Elektronische Vorrichtung nach einem der Ansprüche 1 bis 3, wobei der Sensor ferner einen dritten Temperatursensor, horizontal von der Wärmequelle beabstandet, umfasst und der wenigstens eine Prozessor ferner zum Korrigieren der von den ersten Temperatursensoren gemessenen Temperaturen basierend auf der vom dritten Temperatursensor gemessenen Temperatur ausgebildet ist.

5. Elektronische Vorrichtung nach einem der Ansprüche 1 bis 4, wobei die Blutflussinformation wenigstens ein Element der Gruppe umfassend eine Blutflussgeschwindigkeit, eine Blutflussrichtung, eine Körperkerntemperatur und eine Blutzuckeränderung umfasst.

6. Elektronische Vorrichtung nach Anspruch 5, wobei der wenigstens eine Prozessor ferner ausgebildet ist zum Ermöglichen von einem oder mehreren Elementen der Gruppe umfassend:

   das Schätzen der Blutflussgeschwindigkeit basierend auf dem Bluttemperaturunterschied unter Verwendung eines Schätzmodells, das eine Korrelation zwischen dem Bluttemperaturunterschied und der Blutflussgeschwindigkeit definiert,
   das Schätzen des Wärmestroms basierend auf einem Unterschied zwischen den vom ersten Temperatursensor und vom zweiten Temperatursensor, die auf wenigstens einer Seite der Wärmequelle angeordnet sind, gemessenen Temperaturen, und

das Schätzen der Körperkerntemperatur basierend auf dem geschätzten Wärmestrom.

7. Elektronische Vorrichtung nach Anspruch 5, wobei beim Schätzen der Blutflussgeschwindigkeit der wenigstens eine Prozessor ferner ermöglicht:

das Berechnen eines Unterschieds zwischen der geschätzten Blutflussgeschwindigkeit und einer zu einer Referenzzeit geschätzten Referenzblutflussgeschwindigkeit; und

das Vorhersagen einer Änderung des Blutzuckers im Vergleich zur Referenzzeit basierend auf dem Unterschied zwischen der geschätzten Blutflussgeschwindigkeit und einer Referenzblutflussgeschwindigkeit.

8. Elektronische Vorrichtung nach Anspruch 1, wobei der erste Temperatursensor oder/und der zweite Temperatursensor ein Thermistor ist, oder

wobei die Wärmequelle einen Durchmesser von 1 mm bis 10 mm aufweist, oder

wobei ein Abstand zwischen einer Mitte und den beiden gegenüberliegenden Seiten der Wärmequelle innerhalb von 8,4 mm liegt, oder

wobei ein Abstand zwischen dem Paar von ersten Temperatursensoren und dem Paar von zweiten Temperatursensoren 0,4 mm bis 10 mm beträgt, oder

ferner umfassend ein wärmeisolierendes Material, angeordnet zwischen den ersten Temperatursensoren und zwischen den zweiten Temperatursensoren.

9. Verfahren zum Ermöglichen des Schätzens von Blutflussinformationen durch eine elektronische Vorrichtung, wobei das Verfahren umfasst:

das Steuern (911; 1011) einer Wärmequelle zum Erzeugen von Wärme, während die elektronische Vorrichtung in Kontakt mit einem Benutzer ist, wobei das Steuern der Wärmequelle das Steuern eines Einschaltzeitraums und eines Ausschaltzeitraums der Wärmequelle gemäß einem vorbestimmten Zyklus umfasst;

Messen (912; 1012) von ersten Temperaturdaten unter Verwendung eines Paares erster Temperatursensoren, horizontal voneinander beabstandet an zwei gegenüberliegenden Seiten der Wärmequelle, und Messen (913; 1013) von zweiten Temperaturdaten unter Verwendung eines Paares zweiter Temperatursensoren, vertikal beabstandet vom Paar erster Temperatursensoren in einer Dickenrichtung der elektronischen Vorrichtung, um das Schätzen eines Bluttemperaturunterschieds zwischen den beiden gegenüberliegenden Seiten der Wärmequelle basierend auf den ersten Temperaturdaten und den zweiten Temperaturdaten zu ermöglichen und um das Schätzen von Blutflussinformationen des Benutzers basierend auf dem Bluttemperaturunterschied zu ermöglichen,

wobei die ersten und zweiten Temperaturdaten während des Ausschaltzeitraums der Wärmequelle gemessen werden, der auf den Einschaltzeitraum der Wärmequelle folgt.

10. Verfahren nach Anspruch 9, ferner umfassend:

das Berechnen (914, 915; 1016, 1017) eines ersten Temperaturunterschieds zwischen den vom Paar erster Temperatursensoren gemessenen Temperaturen und eines zweiten Temperaturunterschieds zwischen den vom Paar zweiter Temperatursensoren gemessenen Temperaturen, um das Schätzen des Bluttemperaturunterschieds basierend auf dem ersten Temperaturunterschied und dem zweiten Temperaturunterschied zu ermöglichen.

11. Verfahren nach Anspruch 9 oder 10, ferner umfassend das Messen (1014) einer Temperatur unter Verwendung eines dritten Temperatursensors, horizontal beabstandet von der Wärmequelle,

wobei das Schätzen des Bluttemperaturunterschieds das Korrigieren der von den ersten Temperatursensoren gemessenen Temperaturen basierend auf der vom dritten Temperatursensor gemessenen Temperatur umfasst.

12. Verfahren nach einem der Ansprüche 9 bis 11, wobei das Ermöglichen des Schätzens der Blutflussinformation das Ermöglichen des Schätzens einer Blutflussgeschwindigkeit, enthalten in der Blutflussinformation, basierend auf dem geschätzten Bluttemperaturunterschied unter Verwendung eines Schätzmodells, das eine Korrelation zwischen dem Bluttemperaturunterschied und der Blutflussgeschwindigkeit definiert, umfasst.

**Revendications**

1. Dispositif électronique (100) comprenant :

   un capteur (110) qui comprend une source de chaleur (111), une paire de premiers capteurs de température (112a, 112b) espacés horizontalement l'un de l'autre sur deux côtés opposés de la source de chaleur, et une paire de deuxièmes capteurs de température (113a, 113b) espacés verticalement de la paire de premiers capteurs de température en direction de l'épaisseur du dispositif électronique ; et
   au moins un processeur (120) configuré pour :

   contrôler la source de chaleur afin de générer de la chaleur pendant que le dispositif électronique est en contact avec un utilisateur, dans lequel le contrôle de la source de chaleur comprend le contrôle d'une période de marche et d'une période d'arrêt de la source de chaleur selon un cycle prédéterminé ; et
   recevoir des premières données de température mesurées par la paire de premiers capteurs de température et des deuxièmes données de température mesurées par la paire de deuxièmes capteurs de température afin d'activer l'estimation d'une différence de température du sang entre les deux côtés opposés de la source de chaleur, sur la base des premières données de température et des deuxièmes données de température, et afin d'activer l'estimation d'information de circulation sanguine de l'utilisateur sur la base de la différence de température du sang,
   dans lequel les premières et deuxièmes données de température sont mesurées durant la période d'arrêt de la source de chaleur qui suit la période de marche de la source de chaleur.

2. Dispositif électronique selon la revendication 1, dans lequel ledit au moins un processeur est en outre configuré pour : calculer une première différence de température entre les températures mesurées par la paire de premiers capteurs de température et une deuxième différence de température entre les températures mesurées par la paire de deuxièmes capteurs de température afin d'activer l'estimation de la différence de température du sang sur la base de la première différence de température et de la deuxième différence de température.

3. Dispositif électronique selon la revendication 2, dans lequel ledit au moins un processeur est en outre configuré pour :

   obtenir une fonction objective qui utilise une expression relationnelle entre la première différence de température et la différence de température du sang, et une expression relationnelle entre la deuxième différence de température et la différence de température du sang ; et
   obtenir la différence de température du sang à laquelle la fonction objective est minimisée.

4. Dispositif électronique selon l'une des revendications 1 à 3, dans lequel le capteur comprend en outre un troisième capteur de température espacé horizontalement de la source de chaleur, et ledit au moins un processeur est en outre configuré pour corriger les températures mesurées par les premiers capteurs de température sur la base de la température mesurée par le troisième capteur de température.

5. Dispositif électronique selon l'une des revendications 1 à 4, dans lequel l'information de circulation sanguine comprend au moins un paramètre parmi la vitesse de la circulation sanguine, la direction de la circulation sanguine, la température corporelle centrale et le changement de glycémie.

6. Dispositif électronique selon la revendication 5, dans lequel ledit au moins un processeur est en outre configuré pour activer une ou plusieurs des opérations suivantes :

   estimation de la vitesse de la circulation sanguine sur la base de la différence de température du sang en utilisant un modèle d'estimation qui définit une corrélation entre la différence de température du sang et la vitesse de la circulation sanguine,
   estimation du flux de chaleur sur la base d'une différence entre les températures mesurées par le premier capteur de température et le deuxième capteur de température, qui sont disposés sur au moins un côté de la source de chaleur, et
   estimation de la température corporelle centrale sur la base du flux de chaleur estimé.

7. Dispositif électronique selon la revendication 5, dans lequel, lors de l'estimation de la vitesse de la circulation sanguine, ledit au moins un processeur active en outre :

le calcul d'une différence entre la vitesse estimée de la circulation sanguine et une vitesse de référence de la circulation sanguine estimée à un temps de référence ; et

la prédiction d'un changement de la glycémie par rapport au temps de référence sur la base de la différence entre la vitesse estimée de la circulation sanguine et une vitesse de référence de la circulation sanguine.

**8.** Dispositif électronique selon la revendication 1, dans lequel au moins les premiers capteurs de température ou les deuxièmes capteurs de température sont des thermistances, ou

dans lequel la source de chaleur a un diamètre de 1 mm à 10 mm, ou

dans lequel la distance entre le centre et les deux côtés opposés de la source de chaleur est inférieure à 8,4 mm, ou

dans lequel la distance entre la paire de premiers capteurs de température et la paire de deuxièmes capteurs de température est de 0,4 mm à 10 mm, ou

comprenant en outre un matériau thermiquement isolant disposé entre les premiers capteurs de température et entre les deuxièmes capteurs de température.

**9.** Procédé activant l'estimation d'information de circulation sanguine par un dispositif électronique, le procédé comprenant :

le contrôle (911 ; 1011) d'une source de chaleur afin de générer de la chaleur pendant que le dispositif électronique est en contact avec un utilisateur, dans lequel le contrôle de la source de chaleur comprend le contrôle d'une période de marche et d'une période d'arrêt de la source de chaleur selon un cycle prédéterminé ;

la mesure (912 ; 1012) de premières données de température en utilisant une paire de premiers capteurs de température espacés horizontalement l'un de l'autre sur deux côtés opposés de la source de chaleur, et la mesure (913 ; 1013) de deuxièmes données de température en utilisant une paire de deuxièmes capteurs de température espacés verticalement de la paire de premiers capteurs de température en direction de l'épaisseur du dispositif électronique afin d'activer l'estimation d'une différence de température du sang entre les deux côtés opposés de la source de chaleur, sur la base des premières données de température et des deuxièmes données de température, et afin d'activer l'estimation d'information de circulation sanguine de l'utilisateur sur la base de la différence de température du sang,

dans lequel les premières et deuxièmes données de température sont mesurées durant la période d'arrêt de la source de chaleur qui suit la période de marche de la source de chaleur.

**10.** Procédé selon la revendication 9, comprenant en outre :
le calcul (914, 915 ; 1016, 1017) d'une première différence de température entre les températures mesurées par la paire de premiers capteurs de température et d'une deuxième différence de température entre les températures mesurées par la paire de deuxièmes capteurs de température afin d'activer l'estimation de la différence de température du sang sur la base de la première différence de température et de la deuxième différence de température.

**11.** Procédé selon la revendication 9 ou 10, comprenant en outre la mesure (1014) d'une température en utilisant un troisième capteur de température espacé horizontalement de la source de chaleur,

dans lequel l'estimation de la différence de température du sang comprend la correction des températures mesurées par les premiers capteurs de température sur la base de la température mesurée par le troisième capteur de température.

**12.** Procédé selon l'une des revendications 9 à 11, dans lequel l'activation de l'estimation de l'information de circulation sanguine comprend l'activation de l'estimation d'une vitesse de circulation sanguine, incluse dans l'information de circulation sanguine, sur la base de la différence de température du sang estimée en utilisant un modèle d'estimation qui définit une corrélation entre la différence de température du sang et la vitesse de la circulation sanguine.

# FIG. 1

ELECTRONIC DEVICE ~100

SENSOR ~110

SECOND TEMPERATURE SENSOR ~113a

SECOND TEMPERATURE SENSOR ~113b

FIRST TEMPERATURE SENSOR ~112a

HEAT SOURCE ~111

FIRST TEMPERATURE SENSOR ~112b

PROCESSOR ~120

# FIG. 2

# FIG. 3

FIG. 4

ELECTRONIC DEVICE 400

SENSOR 110

SECOND TEMPERATURE SENSOR 113a

SECOND TEMPERATURE SENSOR 113b

THIRD TEMPERATURE SENSOR 115

FIRST TEMPERATURE SENSOR 112a

HEAT SOURCE 111

FIRST TEMPERATURE SENSOR 112b

PROCESSOR 120

# FIG. 5

# FIG. 6

# FIG. 7A

# FIG. 7B

FIG. 8

SENSOR DEVICE 810

SECOND TEMPERATURE SENSOR 813a

SECOND TEMPERATURE SENSOR 813b

FIRST TEMPERATURE SENSOR 812a

HEAT SOURCE 811

FIRST TEMPERATURE SENSOR 812b

COMMUNICATION INTERFACE 814

ELECTRONIC DEVICE 820

STORAGE 821

COMMUNICATION INTERFACE 822

OUTPUT INTERFACE 823

PROCESSOR 824

# FIG. 9

START

↓

CONTROL HEAT SOURCE TO INDUCE
BLOOD TEMPERATURE DIFFERENCE
AT BOTH SIDES OF HEAT SOURCE ⟋911

↓

MEASURE FIRST TEMPERATURES
BY USING PAIR OF FIRST
TEMPERATURE SENSORS ⟋912

MEASURE SECOND TEMPERATURES
BY USING PAIR OF SECOND
TEMPERATURE SENSORS ⟋913

↓

CALCULATE FIRST
TEMPERATURE DIFFERENCE ⟋914

CALCULATE SECOND
TEMPERATURE DIFFERENCE ⟋915

↓

ESTIMATE BLOOD TEMPERATURE
DIFFERENCE BASED ON FIRST
TEMPERATURE DIFFERENCE AND
SECOND TEMPERATURE DIFFERENCE ⟋916

↓

ESTIMATE BLOOD FLOW
INFORMATION BASED ON BLOOD
TEMPERATURE DIFFERENCE ⟋917

↓

END

# FIG. 10

```
                          ┌─────────┐
                          │  START  │
                          └─────────┘
                               │
                               ▼                    ┌1011
              ┌─────────────────────────────────────┐
              │   CONTROL HEAT SOURCE TO INDUCE      │
              │   BLOOD TEMPERATURE DIFFERENCE       │
              │     AT BOTH SIDES OF HEAT SOURCE     │
              └─────────────────────────────────────┘
                  │            │              │
       ┌1014      ▼    ┌1012   ▼      ┌1013    ▼
┌──────────────────┐ ┌──────────────────┐ ┌──────────────────────┐
│MEASURE THIRD     │ │MEASURE FIRST     │ │MEASURE SECOND        │
│TEMPERATURE       │ │TEMPERATURES      │ │TEMPERATURES          │
│BY USING THIRD    │ │BY USING PAIR OF  │ │BY USING PAIR OF      │
│TEMPERATURE SENSOR│ │FIRST TEMPERATURE │ │SECOND                │
│                  │ │SENSORS           │ │TEMPERATURE SENSORS   │
└──────────────────┘ └──────────────────┘ └──────────────────────┘
         │                    │                      │
         └──────────┐  ┌──────┘                      │
                    ▼  ▼          ┌1015               │
              ┌─────────────────────────┐             │
              │CORRECT FIRST TEMPERATURES│            │
              │BASED ON THIRD TEMPERATURE│            │
              └─────────────────────────┘             │
                        │        ┌1016                ▼        ┌1017
              ┌──────────────────┐       ┌──────────────────────┐
              │CALCULATE FIRST   │       │CALCULATE SECOND      │
              │TEMPERATURE       │       │TEMPERATURE DIFFERENCE │
              │DIFFERENCE        │       │                      │
              └──────────────────┘       └──────────────────────┘
                        │                        │
                        └───────────┬────────────┘
                                    ▼          ┌1018
              ┌──────────────────────────────────┐
              │ESTIMATE BLOOD TEMPERATURE         │
              │DIFFERENCE BASED ON FIRST          │
              │TEMPERATURE DIFFERENCE AND         │
              │SECOND TEMPERATURE DIFFERENCE      │
              └──────────────────────────────────┘
                                    │          ┌1019
              ┌──────────────────────────────────┐
              │ESTIMATE BLOOD FLOW                │
              │INFORMATION BASED ON BLOOD         │
              │TEMPERATURE DIFFERENCE             │
              └──────────────────────────────────┘
                                    │
                                    ▼
                              ┌─────────┐
                              │   END   │
                              └─────────┘
```

EP 4 374 779 B1

FIG. 11

# FIG. 12

1200

1210

FIG. 13

FIG. 14

1400    1410

FIG. 15

FIG. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220128413 A1
- US 20190388031 A1
- US 20180014734 A
- US 20120296224 A1